## Europäisches Patentamt

## European Patent Office

## Office européen des brevets

(19)

(11) Veröffentlichungsnummer: **0 256 142**
A1

(12) **EUROPÄISCHE PATENTANMELDUNG**

Veröffentlicht nach Art. 158 Abs. 3 EPÜ

(21) Anmeldenummer: 87901767.1

(22) Anmeldetag: 22.01.87

Daten der zugrundeliegenden internationalen Anmeldung:

(86) Internationale Anmeldenummer:
PCT/SU87/00011

(87) Internationale Veröffentlichungsnummer:
WO87/04456 (30.07.87 87/17)

(51) Int. Cl.³: **C 12 M 1/40**

(30) Priorität: 22.01.86 SU 4035160

(43) Veröffentlichungstag der Anmeldung:
24.02.88 Patentblatt 88/8

(84) Benannte Vertragsstaaten:
BE CH DE FR GB LI NL SE

(71) Anmelder: VSESOJUZNY
NAUCHNO-ISSLEDOVATELSKY INSTITUT OSOBO
CHISTYKH BIOPREPARATOV
ul. Pudozhskaya 7
Leningrad, 197011(US)

(71) Anmelder: TALLINSKY POLITEKHNICHESKY INSTITUT
ul. Ekhitayate, 5
Tallin, 200026(SU)

(72) Erfinder: TEARO, Eduard Nikolaevich
ul. M. Pasternaka, 3-61
Tallin, 200036(SU)

(72) Erfinder: UUS, Endel Gustavovich
Paplite puiestee, 7-1
Tallin, 200009(SU)

(72) Erfinder: KESTNER, Ado Ilmarovich
ul. Khyarma, 11-29
Tallin, 200036(SU)

(72) Erfinder: PAPPEL, Kaie Eduardovna
ul. Mustamyae, 100-63
Tallin, 200033(SU)

(72) Erfinder: ROOSIMELDER, Lembit Paul-Feodorovich
ul. Retke, 22-21
Tallin, 200034(SU)

(72) Erfinder: PASECHNIK, Vladimir Artemovich
Kondratievsky pr., 19-25
Leningrad, 195108(SU)

(72) Erfinder: SAMARTSEV, Mikhail Alexandrovich
ul. Dimitrova, 16-1-198
Leningrad, 192239(SU)

(72) Erfinder: NAKHAPETIAN, Levon Arutjunovich
ul. Mishina, 12-56
Moscow, 125083(SU)

(72) Erfinder: KAPPO, Jury Vladimirovich
ul. Keskuse, 22-34
Tallin, 200033(SU)

(72) Erfinder: PIKKOV, Lui Martovich
ul. Vilde, 52-115
Tallin, 200034(SU)

(74) Vertreter: Finck, Dieter et al,
Patentanwälte v. Füner, Ebbinghaus, Finck Mariahilfplatz
2 & 3
D-8000 München 90(DE)

(54) **BIOKATALYTISCHER REAKTOR.**

(57) A biocatalytic reactor comprises a column (1) provided with pipes (7, 8) for feeding and discharging the liquid and divided by perforated partitions (2) into sections (3, 4, 5), each of which is provided with pipes (9, 10) for feeding and discharging the catalyst. Arranged in the lower part of the column (1) is a chamber (12) connected to a source of compressed air and connected to the lower section (5) through the perforated partition (2), the adjacent sections (3, 4; 4, 5) being interconnected through liquid overflow devices (14).

EP 0 256 142 A1

## BIOKATALYTISCHER REAKTOR

### Technisches Gebiet

Die vorliegende Erfindung bezieht sich auf die Biotechnologie und genauer auf biokatalytische Reaktoren.

### Stand der Technik

Allgemein bekannt sind biokatalytische Reaktoren, die eine Kolonne mit einer in ihrem unteren Teil befindlichen Perforierscheidewand aufweisen. Die Kolonne ist im oberen und unteren Teil mit Rohrstutzen zur Zu- und Abfuhr von Flüssigkeit (einer Substratlösung) versehen. Im oberen Teil der Kolonne ist ein Stutzen für deren Einfüllen mit einem Katalysator und im unteren Teil - für dessen Austragen vorgesehen.

Bei solchen Reaktoren bleibt die Katalysatorschicht unbeweglich. Bei der Durchströmung einer trüben bzw. kolloidalen Lösung durch die Katalysatorschicht erfolgt allmähliche Ansammlung von Fällprodukt in den Poren und zwischen den Körnern des Katalysators, was zur Verminderung der Geschwindigkeit des Durchströmens der Lösung sowie zur Bildung von Kanälen führt. Das alles vermindert die bezogene Leistung vom Katalysator (Leistung pro Einheit der Katalysatormasse), schafft die Voraussetzungen für seine Impfung mit Mikroflora und verursacht die Notwendigkeit der Durchführung systematischer Durchspülungen des Katalysators, was, seinerseits, die Leistung der Kolonne herabsetzt.

Darüber hinaus ist eine Konstruktion biokatalytischen Reaktors bekannt, der eine Kolonne enthält, die Rohrstutzen für Zu- und Abfuhr von Flüssigkeit aufweist und durch über die ganze Fläche perforierte Scheidewände in Sektionen eingeteilt ist, von denen jede einen Rohrstutzen für die Zu- und Abfuhr des Katalysators aufweist. Der Rohrstutzen für die Katalysatorzufuhr ist im oberen Teil der Kolonne angeordnet und dient zur Zuführung vom Katalysator in die obere Sektion. Die benachbarten Sektionen kommunizieren miteinander durch Überlaufrohre, mit deren Hulfe di

Suspension des Katalysators sich allmählich von oben nach unten bewegt. Der verbrauchte Katalysator wird periodisch durch den in der unteren Sektion der Kolonne befindlichen Rohrstutzen entfernt. Die Flüssigkeit (Substratlösung) wird unter Druck durch den Rohrstutzen einem Behälter zugeführt, der sich im unteren Teil der Kolonne unter der perforierten Scheidewand der unteren Sektion befindet, fliesst durch die Perforation in der Scheidewand in die untere Sektion und bewegt sich allmählich über die Sektionen von unten nach oben, wobei sie durch den Rohrstutzen entnommen wird, der sich über der oberen Sektion befindet (s. beispielsweise FR-Anmeldung Nr. 2400386 IPK B 01 j 8/20, veröffentlicht am 16.03.79). Im Reaktor wird also eine Gegenstrombewegung von Flüssigkeit und Katalysator erreicht, wobei durch die Bewegung der Flüssigkeit von unten nach oben der Katalysator in den Sektionen in einem scheinflüssigen Zustand gehalten wird, was es ermöglicht, Flüssigkeit mit Katalysatorteilchen zu vermischen, die Bildung von Kanälen zu verhindern und die Entstehung eines Fällproduktes in den Poren und zwischen den Körnern des Katalysators zu beschränken.

Das in diesem Reaktor erreichbare Vermischen der Teilchen des Katalysators macht den Reaktor leistungsfähiger im Vergleich zum obengenannten, aber seine Leistung ist durch die Geschwindigkeit des Durchströmens der Flüssigkeit durch die Wirbelschicht eingeschränkt, weil bei einer beträchtlichen Geschwindigkeit die Wirbelschicht zerstört wird.

Offenbarung der Erfindung

Der Erfindung liegt die Aufgabe zugrunde, einen biokatalytischen Reaktor zu entwickeln, bei dem das Vermischen des Katalysators und der Überlauf der Flüssigkeit aus der einen Sektion in die andere (aus der oberen in die untere) derart vor sich geht, dass der Nutzeffekt des Katalysators und somit die Leistung des Reaktors erhöht werden.

Diese Aufgabe wird dadurch gelöst, dass in einem biokatalytischen Reaktor, der eine Kolonne mit Rohrstutzen für die Zu- und Abfuhr von Flüssigkeit aufweist, die durch über die ganze Fläche perforierte Scheidewände in Sektionen eingeteilt ist, von denen jede Rohrstutzen für die Zu- und Abfuhr des Katalysators aufweist, erfindungsgemäss, im unteren Teil der Kolonne eine Kammer ausgeführt ist, die mit einer Druckgasquelle kommuniziert und von der unteren Sektion durch die perforierte Scheidewand getrennt ist, wobei die benachbarten Sektionen durch Überlaufeinrichtungen miteinander verbunden sind.

Zweckmässigerweise enthält jede Überlaufeinrichtung einen Zylinder, der ausserhalb der Kolonne längs der oberen Sektion angeordnet ist und mit oberem und unterem Teil dieser Sektion kommuniziert, eine Röhre, die im Zylinder untergebracht ist und dessen offenes Ende oberhalb der Sektionsmitte angeordnet, das andere Ende aber mit dem unteren Teil der unteren Sektion verbunden ist, und ein Filterelement, das in der oberen Sektion in der Zone der Anschliessung des erwähnten Zylinders liegt.

Solch eine Ausführung der Überlaufeinrichtung empfiehlt sich für die biokatalytischen Reaktoren mit relativ geringen Abmessungen.

Jede Überlaufeinrichtung kann einen von oben geöffneten Zylinder der in der oberen Sektion angeordnet ist und in dessen Innerem eine Röhre mit Längsschlitzen für das Durchströmen von Flüssigkeit und Gas untergebracht ist, die mit ihrem unteren Ende mit dem unteren Teil der benachbarten unteren Sektion kommuniziert, und ein Filterelement enthalten, das in Form einer perforierten Einfassung ausgeführt ist, die von oben geschlossen ist und sich auf die perforierte Scheidewand stützt, wobei das obere offene Ende der Röhre oberhalb der Einfassung liegt.

Solche Ausführung der Überlaufeinrichtung eignet sich zweckmässigerweise für die Reaktoren beliebiger Abmessungen, wobei in nur einer Sektion mehrere Filtrierelemente angeordnet werden können. Bei solcher Anordnung erleichtert sich die Auswechselung der Filterelemente während der Reparierung.

-4-

parierung.

Zweckmässigerweise enthält jede Überlaufeinrichtung zwei koaxial angeordnete Einfassungen, die innerhalb der oberen Sektion untergebracht sind, die innere Einfassung hat dabei in ihrem unteren Teil durchgehende Öffnungen zum Überströmen der Flüssigkeit, und in dieser ist eine Röhre angeordnet, deren oberes Ende oberhalb der äusseren Einfassung liegt und das untere Ende mit dem unteren Teil der benachbarten unteren Sektion kommuniziert, dabei ist an der Röhre eine Strömungshaube angeordnet, die in Form von zwei Kegeln mit einer gemeinsamen Grundfläche ausgeführt und mit einem Zwischenraum in bezug auf die unteren Stirnflächen der Einfassungen angeordnet ist, und auf der Seite der oberen Stirn der inneren Einfassung ist eine Strömungshaube mit einem Ringspalt in bezug auf diese angeordnet, die mit ihrer Spitze nach oben zeigt.

Solch eine Ausführung der Überlaufeinrichtung empfiehlt sich für Reaktoren, die für die Arbeit mit stark verschmutzten Flüssigkeiten vorbestimmt sind, die feindisperse Teilchen enthalten, die die Filterelemente verstopfen können.

Nicht weniger zweckmässig ist es, dass in der oberen Sektion der Kolonne ein trichterförmiges Element untergebracht ist, das ihren Querschnitt überdeckt und mit seinem sich verjüngenden Teil nach unten zeigt, wobei sein offenes unteres Ende im unteren Teil der oberen Sektion liegt und auf der konischen Wandung tangential angeordnet Öffnungen für die Gasabfuhr ausgeführt sind.

Die Anordnung des trichterförmigen Elementes ist für die Arbeit mit Flüssigkeiten vorbestimmt, die zur plötzlichen Schaumbildung unter der Einwirkung barbotierenden Gases geneigt sind, und bewirkt ein Schaumbrechen.

Es ist wünschenswert, dass die Öffnungen der Scheidewände die Form eines Kegelstumpfes haben, der mit seiner geringeren Grundfläche nach unten zugewandt ist, dabei muss die Perforationsfläche jeder Scheidewand 0,1 bis 1,0% der Fläche des lichten Querschnitts der Kolonne betragen.

Solche Ausführung der Perforation der Scheidewände ermöglicht die Minimisierung vom Gasverbrauch unter Erhaltung der vorgegebenen Intensität des Vermischens des Katalysators, beugt dem Durchgehen der Katalysatorteilchen durch die Öffnungen und dem Verstopfen der letzteren vor.

Die erfindungsgemässen biokatalytischen Reaktoren ermöglichen bei ihrer ziemlich einfachen konstruktiven Ausführung, die biokatalytischen Prozesse zu intensivieren, den Nutzeffekt der Biokatalysatoren zu erhöhen, als Substrate trübe und kolloidale Lösungen, als Katalysatoren aber Fermente und Zellen, die sowohl auf harten mineralischen wie auch auf weichen organischen Trägern immobilisiert sind, und zur Barbotage sowohl inerte als auch aktive Gasmedien zu verwenden.

Kurzbeschreibung der Zeichnungen

Nachstehend im Text wird die Beschreibung konkreter Beispiele der Ausführung der Erfindung unter Bezugnahme auf die beigelegten Zeichnungen angeführt, wobei es zeigt:

Fig. 1 schematische Darstellung des erfindungsgemässen biokatalytischen Reaktors, in Längsschnitt, mit einer Überlaufeinrichtung, die ausserhalb der Kolonne montiert ist;

Fig. 2 einen Schnitt II-II der Fig. 1;

Fig. 3 schematische Darstellung des biokatalytischen Reaktors mit einer Überlaufeinrichtung, die auf der Scheidewand montiert ist, in Längsschnitt;

Fig. 4 eine Einheit A der Fig. 3 in vergrössertem Masstab, in Längsschnitt;

Fig. 5 schematische Darstellung des biokatalytischen Reaktors mit einer Überlaufeinrichtung, die innerhalb der Sektion montiert ist, in Längsschnitt;

Fig. 6 eine Einheit B der Fig. 5 in vergrössertem Masstab, in Längsschnitt.

Beste Ausführungsbeispiele der Erfindung

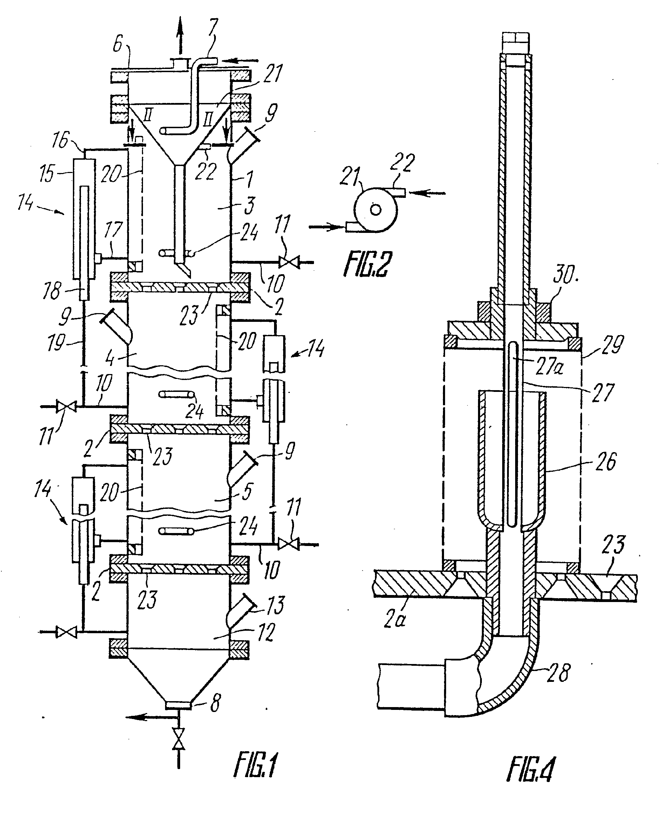

Der biokatalytische Reaktor enthält eine Kolonne 1 (Fig. 1), die durch perforierte Scheidewände 2 in Sektionen 3, 4, 5 eingeteilt ist. An die Kolonne 1 ist über einen Deckel 6 ein Rohrstutzen 7 angeschlossen, der zur Zuführung der Flüssigkeit (Substratlösung) dient, und unten an der Kolonne ist ein Rohrstutzen 8 für die Abführung der Flüssigkeit, die durch die Kolonne durchgegangen ist, vorgesehen. Jede Sektion hat in ihrem oberen Teil ein Rohrstutzen 9 für die Einladung des Katalysators und im unteren Teil ein Rohrstutzen IO für die Ausladung des Katalysators, der durch eine Sperreinrichtung 11 überdeckt wird. Im unteren Teil der Kolonne 1 ist eine Kammer 12 ausgeführt, die durch einen Rohrstutzen 13 mit einer Druckgasquelle (nicht angedeutet) kommuniziert. Die Kammer 12 trennt sich von der Sektion 5 durch die perforierte Scheidewand 2. Das Überströmen von Flüssigkeit aus jeder oberen Sektion in die benachbarte untere Sektion ist durch eine Überlaufeinrichtung 14 verwirklicht.

Die Überlaufeinrichtungen 14, die die jeweilige obere Sektion mit der benachbarten unteren Sektion verbinden, sind identisch ausgeführt, und deshalb bezieht sich alles in der weiteren Beschreibung, was die eine derselben betrifft, auch auf die anderen. Jede Überlaufeinrichtung 14 enthält einen Zylinder 15, der ausserhalb der Kolonne 1 längs der oberen Sektion 3 angeordnet ist; der Zylinder 15 kommuniziert durch Rohrleitungen 16 und 17 mit der oberen Sektion 3 in ihrem oberen bzw. unteren Teil. Innerhalb des Zylinders 15 ist eine Röhre 18 untergebracht, deren offenes Ende sich über der Mitte der Sektion 3 in der Höhe des vorgegebenen Flüssigkeitsspiegels in dieser Sektion befindet. Das andere Ende dieser Röhre kommuniziert durch eine Rohrleitung 19 mit der unteren Sektion 4 über einen Rohrstutzen 10. Das Anschalten der Rohrleitung 19 an den Rohrstutzen 10 erfolgt zwischen der Sektion 4 und der Sperreinrichtung 11. Für die Zurückhaltung des Katalysators in der Sektion 3 ist in der Anschaltzone der Rohrleitung 16 und 17 ein Filterelement 20 vorgesehen, als welches in dem beschriebenen Beispiel das Netz mit dem Zellenmass kleiner als Durchmesser der Teilchen des Katalysators benutzt ist. In den biokatalytischen Reaktoren werden die Überlaufeinrichtungen wie oben beschrieben relativ geringerer Abmessungen eingestellt.

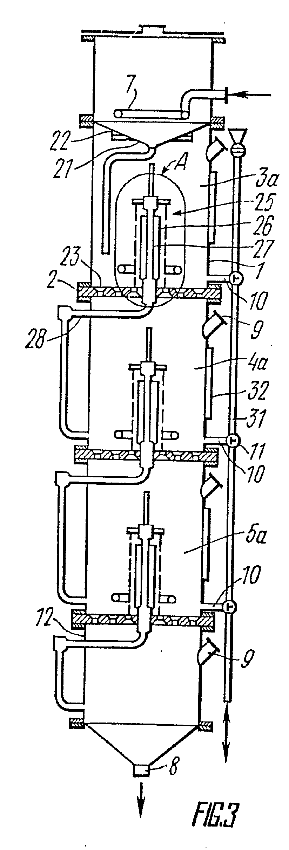

Im oberen Teil der oberen Sektion 3 ist ein Trichterelement 21 (in der weiteren Beschreibung Trichter 21 genannt) montiert. Der Trichter 21 überdeckt mit ihrem breiten Teil den Querschnitt der Sektion 3, und dessen verjüngter Teil liegt im unteren Teil der Sektion 3 in einem Abstand von der Scheidewand 2, der für das Ausströmen der Flüssigkeit aus dem Trichter 21 ausreichend ist, die in diesen durch den Rohrstutzen 7 einströmt. Der Trichter 21 hat auf ihrer konischen Wandung · tangential angeordnete Öffnungen, an welche Rohrstutzen 22 (Fig. 2) für den Gasdurchgang angeschlossen sind. Diese Öffnungen und Rohrstutzen 22 dienen für das Schaumbrechen im Falle einer plötzlichen Bildung von Schaum in der oberen Sektion 3.

Zur Optimisierung der Bedingungen für den Gasdurchgang durch alle Sektionen 3, 4, 5 unter Erhaltung der vorgegebenen Intensität des Vermischens vom Katalysator, Vorbeugung des Durchgehens der Teilchen des Katalysators durch die Öffnungen 23 (Perforation) in den Scheidewänden 2 und des Verstopfens der letzteren beträgt die Fläche der Perforation jeder Schwidewand 2 0,1 bis 1,0% der Fläche des Querschnitts der Kolonne, und die Öffnungen 23 der Scheidewände 2 weisen die Form eines Kegelstumpfes auf, der mit seiner geringeren Grundfläche nach unten gewandt ist. Diese Form bewirkt eine intensive Zirkulation des Katalysators mit der Flüssigkeit über der Scheidewand.

Zur Aufrechterhaltung der Solltemperatur in jeder Sektion 3, 4, 5 sind Wärmeaustauscherelemente 24 montiert, die eine beliebige Bauart haben können, die zu diesem Zweck geeignet ist.

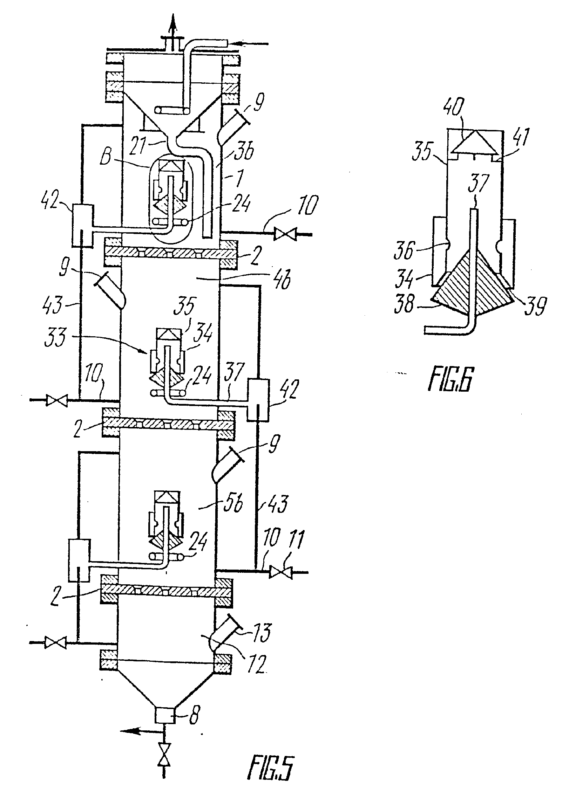

Bei den Reaktoren, die einen relativ grossen Durchmesser haben, ist jede Überlaufeinrichtung 25 (Fig. 3), die eine obere Sektion 3a mit der benachbarten unteren Sektion 4a verbindet, wie folgt ausgeführt. Die Überlaufeinrichtung 25 enthält einen von oben offenen Zylinder 26 (Fig. 4), der in der oberen Sektion 3a (Fig. 3) montiert ist. Das offene Ende vom Zylinder 26 liegt in einer Höhe, die dem vorgegebenen Flüssigkeitsspiegel in der Sektion 3a entspricht. Innerhalb des Zylinders 26 geht eine Röhre 27 mit Längsschlitzen 27a (Fig. 4), die zum Durchleiten von Flüssigkeit und Gas in die Röhre 27 vorbestimmt sind. Das untere Ende der Röhre 27 kommuniziert durch eine Rohrleitung 28 mit dem unteren Teil der unteren Sektion 4a. Um den Zylinder 26 ist ein Filterelement in Form einer perforierten Einfassung 29 aufgestellt, das oben mit einem Deckel 30 abgeschlossen ist und sich auf die Scheidewand 2a stützt. Das offene Ende der Röhre 27 geht durch den Deckel 30 hindurch. In diesem Beispiel sind die Rohrstutzen 10 (Fig. 3) zur Abführung des Katalysators durch die Sperreinrichtungen 11 an eine gemeinsame Rohrleitung 31 angeschaltet, was es ermöglicht, den Katalysator aus jeder Sektion in eine beliebige andere Sektion umzuladen. Die Seitenwand jeder Sektion 3a, 4a, 5a hat eine Montagelücke 32, die es ermöglicht, den Zustand des Filterelementes zu prüfen und es gegebenenfalls zu ersetzen. Im übrigen ist der auf Fig. 3 gezeigte Reaktor analog zum oben beschriebenen Reaktor ausgeführt.

In den Reaktoren, die zur Arbeit mit trüben und verunreinigten Flüssigkeiten vorgesehen sind, enthält eine jede Überlaufeinrichtung 33 (Fig. 5) zwei koaxial angeordnete Einfassungen 34 und 35. Das obere offene Ende der äusseren Einfassung 34 befindet sich unter dem Flüssigkeitsspiegel in der Sektion 3b (Fig. 5) und dient zur Strombildung der Flüssigkeit. Die innere Einfassung 35 hat in ihrem unteren Teil durchgehende Öffnungen 36 (Fig. 6), die die Innenräume der Einfassungen 34 und 35 miteinander verbinden. Innerhalb der Einfassung 35 ist eine Röhre 37 untergebracht, deren oberes offenes Ende oberhalb der äusseren Einfassung angeordnet ist und deren unteres Ende mit dem unteren Teil der benachbarten unteren Sektion 4b kommuniziert. An der Röhre 37 ist eine Strömungshaube 38 befestigt, die als zwei Kegel ausgeführt ist, die mit ihren

-9-

Grundflächen zueinander gewandt sind. Die Strömungshaube 38 ist mit einem Zwischenraum 39 in bezug auf die unteren Stirnen der Einfassungen 34 und 35 montiert.

Der Zwischenraum 39 zwischen dem oberen Kegel der Strömungshaube 38 und den Stirnen der Einfassungen 34 und 35 dient zur Abführung der Teilchen vom Katalysator aus dem Innenraum zwischen den Einfassungen 34 und 35, und der untere Kegel der Strömungshaube 38 - zur Vorbeugung des Geratens von Gas in den Zwischenraum zwischen den Einfassungen 34 und 35.

Im oberen Teil der inneren Einfassung 35 ist eine Strömungshaube 40 mit einem Ringspalt 41 zwischen ihr und der Einfassung 35 montiert. Die Strömungshaube 40 stellt einen Kegel dar, der mit seiner Spitze nach oben gewandt ist, er verhindert das Geraten der Teilchen des Katalysators in die Röhre 37, die mit dem Schaum mitgerissen werden.

Im zu betrachtenden Beispiel gelangt die Flüssigkeit aus der Röhre 37 der Überlaufeinrichtung 33 (Fig. 5) in eine Kammer 42, woraus die Flüssigkeit über eine Rohrleitung 43 in den Rohrstutzen 10 im unteren Teil der benachbarten unten liegenden Sektion fliesst. Die Kammer 42 ist zur eventuellen Anordnung eines pH-Gebers und Einführung eines Alkali oder einer Säure für das Titrieren vorgesehen, was im Falle der Prozesse notwendig ist, bei denen sich der pH-Wert des Mediums ändert. Im übrigen ist der in Fig. 5 dargestellte Reaktor ähnlich den oben beschriebenen ausgeführt.

Der biokatalytische Reaktor, der in Fig. 1 gezeigt ist, arbeitet wie folgt.

Zum Ingangsetzen des Reaktors werden alle Rohrstutzen 9 für die Einladung des Katalysators geöffnet und die Gaszufuhr über den Rohrstutzen 13 eingeschaltet. Danach wird in die untere Sektion 5 durch den Rohrstutzen 9 eine Katalysatorsuspension in der Substratlösung (in der weiteren Beschreibung als Substrat genannt) in einer Menge, die zur Arbeit dieser Sektion vorbestimmt ist, einge-

gossen. Die Menge des Katalysators und des Substrats in der Sektion 5 wird durch die Höhe der Anordnung der Röhre 18 zum Abfluss von Flüssigkeit in der Überlaufeinrichtung 14 bestimmt. Dann wird der Rohrstutzen 9 der unteren Sektion 5 verschlossen, und es wird mit allen höher gelegenen Sektionen 4, 3 gleichartig gehandelt. Das der Kammer 12 über den Rohrstutzen 13 zugeführte Gas strömt allmählich durch die Öffnungen 23 der Scheidewände 2 der Sektionen 5, 4, 3 durch, indem es den in diesen befindlichen Katalysator in scheinflüssigen Zustand überführt, und verlässt den Reaktor durch den Rohrstutzen 22 des Trichters 21, der die Rolle eines Schaumbrechers spielt. Es werden Wärmeaustauscherelemente 24 eingeschaltet und die im Reaktor befindlichen Katalysator und Substrat werden thermostatiert.

Darauffolgend beginnt man mit ununterbrochener Substratzufuhr in die Kolonne 1 durch den Rohrstutzen 7, wobei der Verbrauch an Substrat durch die Kinetik der durchzuführenden Reaktion bestimmt wird. Das Substrat fliesst über die Wände des Trichters ab und gelangt in die obere Sektion 3. Das Substrat kommt mit scheinflüssigem Katalysator in Wechselwirkung und gelangt durch das Filterelement 20 über die Rohrleitung 17 in den Zylinder 15, in welchem die Röhre 18 zum Abfluss von Substrat untergebracht ist; das obere offene Ende dieser Röhre bestimmt den Spiegel von Substrat in der Sektion 3, das überschüssige Substrat strömt über die Röhre 18 und Rohrleitung 19 in die unten liegende Sektion 4 , wo es mit einer neuen Portion von Katalysator in Wechselwirkung kommt, danach wiederholt sich das Spiel. Aus der letzten Sektion 5 gerät das Substrat in die Kammer 12 und verlässt den Reaktor durch den Rohrstutzen 8.

Ein Kurzstillstand des Reaktors ohne Ausladung von Katalysator ist möglich, wenn man die Gaszufuhr in den Reaktor durch den Rohrstutzen 13 einstellt und die Dichtigkeit jeder Sektion 3, 4, 5 der Kolonne beibehält. Die Erwärmungselemente 24 müssen dabei abgeschaltet werden, damit eine mögliche Überhitzung von Katalysator und Substrat ausgeschlossen ist. Um den Reaktor wiederum in Betrieb zu setzen, ist es in diesem Fall genügend, wenn man mit der Zuführung von Gas und Substrat in den Reaktor beginnt.

Die Zuführung von Substrat in den Reaktor kann zu jeder Zeit ohne Störung hydrodynamischer und Wärmeverhältnisse des Reaktors eingestellt werden.

Bei der Arbeit des Reaktors kommt es zu keinen Überströmen von Katalysator aus einer Sektion in die andere. Die Austragung des Katalysators aus den Sektionen nach seiner Abnutzung wird durch die Rohrstutzen 10 bei offener Sperreinrichtung 11 bewerkstelligt.

Der biokatalytische Reaktor, gezeigt in Fig. 3, arbeitet im wesentlichen analog zum oben beschriebenen. Der Aufbau seiner Überlaufeinrichtung 25 ermöglicht es, die Reaktoren mit einer mehr entwickelten Oberfläche der Filterelemente zu schaffen, was besonders wichtig für die Reaktoren mit relativ grossen Durchmessern und Leistung ist. Verbessert sind auch die Bedingungen für Auswechselung der Filterelemente im Laufe des Betriebes.

Diese Einrichtung arbeitet wie folgt. Das in die Sektion 3a gelangende Substrat wird mit scheinflüssigem Katalysator umgesetzt, dann geht durch die filtrierende Einfassung 29 durch, fliesst über den Rand des Zylinders 26 und weiterhin gelangt durch die Längsschlitze 27a über die Rohrleitung 28 in die unten liegende Sektion 4a. Zum Druckausgeleich zwischen den Innenräumen der Einfassung 29 und der Sektion ist die Röhre 27 nach oben über den Spiegel von Substrat in der Sektion 3a herausgeführt und kommuniziert über die Schlitze 27a mit dem Gasraum der Sektion 3a.

Im biokatalytischen Reaktor, der in Fig. 5 dargestellt ist, wird der Katalysator vom Substrat in der Überlaufeinrichtung 33 abgetrennt, die es ermöglicht, mit den Substraten zu arbeiten, die feindisperse oder kolloidale Teilchen enthalten, oder mit Substraten, worin ähnliche Teilchen während der Arbeit gebildet werden. Zirkulationsströme der

Suspension von Katalysator mit Substrat, die in der Sektion 3b beim Durchgehen von Gas durch diese entstehen, gelangen in den Hohlraum zwischen den Zylindereinfassungen 34 und 35 in Richtung von oben nach unten.

Dabei kehrt der sich in diesem Hohlraum abscheidende Katalysator durch den Zwischenraum 39 über die Strömungshaube 38 in die Sektion 3b zurück, und das teilweise von diesem befreite Substrat dringt durch die Öffnungen 36 ins Innere der Einfassung 35 ein, wo es sich endgültig vom Katalysator befreit wird und durch die Röhre 37 in die unten liegende Sektion 4b gelangt.

Die Menge von Substrat, das in diesem Falle dem Reaktor zugeführt wird, wird mit den Bedingungen für die Abscheidung des Katalysators in der inneren Zylinderfassung 35 bestimmt.

Der erfindungsgemässe biokatalytische Reaktor besitzt im Vergleich zu den bekannten Apparaten (Aufsatzapparaten) einen geringen hydraulischen Widerstand selbst bei Anwendung feindisperser Katalysatoren. Er kann eine beliebige Zahl der Sektionen aufweisen. Durch intensive Vermischung des Katalysators mit dem Substrat werden Oberflächendiffusions-Widerstände des Stoffüberganges maximal aufgehoben, die Ablagerung von Fällprodukten auf den Körnern des Katalysators wird beseitigt, was es ermöglicht, auf Substraten zu arbeiten, die feindisperse oder kolloidale Teilchen enthalten. Alles Obenaufgezählte gerade vergrössert spezifische Leistung des Katalysators.

Versuchsmuster der erfindungsgemässen biokatalytischen Reaktoren wurden in zwei Masstäben hergestellt, mit einem Arbeitsvolumen der Sektionen von 2,5 und 32 1 mit einem Innendurchmesser der Kolonnen von 140 und 300 mm, mit einer Gesamthöhe von 2300 bzw. 7000 mm. Jeder Reaktor enthielt vier Sektionen. Die Versuche haben ergeben, dass eine Änderung des Masstabes die Effektivität der Prozessführung nicht beeinflusst.

Diese Reaktoren zeigten eine hohe Wirksamkeit in Prozessen der Hydrolyse eines 20-30%igen Stärkesirups durch immobilisierte Glukoamylase, der Herstellung eines Gluko-
se-Fruktose-Sirups mit immobilisierter Glukoseisomerase,
der Hydrolyse von Saccharose durch immobilisierte Invertase, der Hydrolyse von Laktose in Vollmolke durch immobilisierte Laktase, der Herstellung von L-Methionin aus
Azetyl-DL-Methionin mit immobilisierter Aminoazylase, der
Herstellung der 6-Aminopenizillansäure aus Benzylpenizillin mit immobilisierter Betalaktamase. Die Leistung des
Reaktors, die in Kilogrammen Produkt, bezogen auf 1 kg
Trockenkatalysator, pro eine Stunde ausgedrückt ist, war
bei einem bestimmten Umwandlungsgrad des Substrats für
alle untersuchten Verfahren höher als für die Kolonnenreaktoren mit einer fixierten Katalysatorschicht. Als Katalysatoren wurden sowohl Fermente, die nach chemischem Verfahren auf einem Mineralträger (Silochrom) immobilisiert
sind, als auch Fermente, die in Teilchen von Polyakrylamidgel eingefügt sind, angewendet; in allen Fällen war
die Zerstörung des Trägermaterials gering.

Industrielle Anwendbarkeit

Am zweckmässigsten ist der erfindungsgemässe biokatalytische Reaktor in Nahrungs-, pharmazeutischer und chemischer Industrie zu verwenden, zur Durchführung von Prozessen, in denen als Katalysatoren immobilisierte Fermente oder Zellen verwendet werden, das heisst Fermente oder
Zellen, die auf Teilchen dispersen Trägers fixiert oder
in Teilchen von Gel eingeführt sind.

0256142

-14-

PATENTANSPRÜCHE

1. Biokatalytischer Reaktor, der eine Rohrstutzen (7, 8) zur Zu- und Abfuhr von Flüssigkeit aufweisende Kolonne (1) enthält, die durch über die ganze Fläche perforierte Scheidewände (2) in Sektionen (3, 4, 5) eingeteilt ist, von denen jede Rohrstutzen für die Zu- und Abführung von Katalysator aufweist, dadurch g e k e n n z e i c h - n e t , dass im unteren Teil der Kolonne (1) eine Kammer (12) ausgeführt ist, die mit einer Druckgasquelle kommuniziert und von der unteren Sektion (5) durch die perforierte Scheidewand abgetrennt ist, wobei die benachbarten Sektionen (3, 4; 4, 5) durch Überlaufeinrichtungen für Flüssigkeit miteinander verbunden sind.

2. Biokatalytischer Reaktor nach Anspruch 1, dadurch g e k e n n z e i c h n e t , dass jede Überlaufeinrichtung (14) einen Zylinder (15), der ausserhalb der Kolonne (1) längs der oberen Sektion (3) angeordnet ist und mit dem oberen und unteren Teil dieser Sektion kommuniziert, eine Röhre (18), die im Zylinder (15) montiert ist, deren offenes Ende über der Mitte der Sektion (3) liegt und das andere Ende mit dem unteren Teil der unteren Sektion (4) kommuniziert, sowie ein Filterelement (20) aufweist, das in der oberen Sektion (3) in der Zone der Anschliessung des erwähnten Zylinders (15) liegt.

3. Biokatalytischer Reaktor nach Anspruch 1, dadurch g e k e n n z e i c h n e t , dass jede Überlaufeinricht- ung (25) einen von oben offenen Zylinder (26), der in der oberen Sektion (3a) untergebracht ist und in dessen Innerem eine Röhre (27) mit Längsschlitzen (27a) zum Durchströmen von Flüssigkeit und Gas gelegen ist, die mit ihrem unteren Ende mit dem unteren Teil der benachbarten unteren Sektion (4a) kommuniziert, und ein Filterelement, das in Form einer perforierten Einfassung (29) ausgeführt ist, die von oben geschlossen ist und sich auf die perforierte Scheidewand (2a) stützt, enthält, wobei das offene Ende der Röhre (27) oberhalb der Einfassung (29) angeordnet ist.

4. Biokatalytischer Reaktor nach Anspruch i, dadurch

g e k e n n z e i c h n e t , dass jede Überlaufeinrichtung (33) zwei koaxial angeordnete Einfassungen (34, 35)
enthält, die in der oberen Sektion (3b) untergebracht
sind, wobei die innere Einfassung (35) in ihrem unteren
Teil durchgehende Öffnungen (36) zum Überströmen der Flüssigkeit aufweist und in dieser eine Röhre (37) eingestellt
ist, deren oberes Ende über der äusseren Einfassung (34)
liegt und das untere Ende mit dem unteren Teil der benachbarten unteren Sektion (4b) kommuniziert, und an der Röhre
(37) eine Strömungshaube (38) in Form von zwei Kegeln mit
gemeinsamer Grundfläche angeordnet ist, wobei zwischen ihr
und den unteren Stirnen der Einfassungen (34, 35) ein Zwischenraum (39) vorgesehen ist, und auf der Seite der oberen Stirn der inneren Einfassung (35) eine kegelförmige
Strömungshaube (40) mit einem Ringspalt (41) zu dieser angeordnet und mit ihrer Spitze nach oben gewandt ist.

5. Biokatalytischer Reaktor nach Anspruch 1 bis 4,
dadurch g e k e n n z e i c h n e t , dass in der oberen
Sektion der Kolonne (1) ein Trichterelement (21) eingestellt ist, das deren Querschnitt überdeckt und mit ihrem
sich verjüngenden Teil nach unten gewandt ist, wobei dessen offenes unteres Ende im unteren Teil der oberen Sektion
(3) liegt, und auf der konischen Wandung des Trichterelementes tangential angeordnete Öffnungen für die Gasabfuhr
vorgesehen sind.

6. Biokatalytischer Reaktor nach Anspruch 1, dadurch
g e k e n n z e i c h n e t , dass die Öffnungen (23) der
Scheidewände (2) die Form eines Kegelstumpfes haben, der
mit seiner geringeren Grundfläche nach unten gewandt ist,
wobei die Perforationsfläche jeder Scheidewand (2) 0,1 bis
1,0% der Fläche des lichten Querschnitts der Kolonne (1)
beträgt.

*FIG.1*

*FIG.2*

*FIG.4*

FIG.3

FIG.5

FIG.6

# INTERNATIONAL SEARCH REPORT

International Application No PCT/SU 02 56 142

## I. CLASSIFICATION OF SUBJECT MATTER (if several classification symbols apply, indicate all)

According to International Patent Classification (IPC) or to both National Classification and IPC

IPC⁴: C 12 M 1/40

## II. FIELDS SEARCHED

Minimum Documentation Searched

| Classification System | Classification Symbols |
|---|---|
| IPC⁴ | C 12 M 1/40 |

Documentation Searched other than Minimum Documentation
to the Extent that such Documents are Included in the Fields Searched

## III. DOCUMENTS CONSIDERED TO BE RELEVANT

| Category | Citation of Document, with indication, where appropriate, of the relevant passages | Relevant to Claim No. |
|---|---|---|
| A | EP, A2, 0042306, (Exxon Research and Engineering Company), 23 December 1981 (23.12.81), see figures 1,2, the abstract | 1 |
| A | EP, A2, 0137688, (IMPERIAL CHEMICAL INDUSTRIES PLC), 17 April 1985 (17.04.85), see page 12, figure 1 | 1 |
| A | FR, A1, 2400386, (STAMICARBON B.V.), 16 March 1979 (16.03.79), see pages 10,11, figures 1,2 | 1 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 20 March 1987 (20.03.87) | 30 April 1987 (30.04.87) |
| International Searching Authority | Signature of Authorized Officer |
| ISA/SU | |

Form PCT/ISA/210 (second sheet) (January 1985)